# EUROPEAN PATENT SPECIFICATION

(11) **EP 3 115 051 B1**
(45) Date of publication and mention of the grant of the patent: **27.04.2022**
(21) Application number: 15757899.8
(22) Date of filing: 04.03.2015
(51) Int. Cl.: A61K 31/497, A61K 31/498, A61K 31/506, A61P 33/10, C07D 241/24, C07D 241/44, C07D 401/12, C07D 403/12, C07D 413/12, C07D 417/12

(54) **ENDOPARASITE CONTROL AGENT**
ENDOPARASITENBEKÄMPFUNGSMITTEL
AGENT DE LUTTE CONTRE LES ENDOPARASITES

(30) Priority: 05.03.2014 JP 2014042878
(43) Date of publication of application: 11.01.2017
(73) Proprietor: The University of Tokyo, Tokyo 113-8654 (JP); Nihon Nohyaku Co., Ltd., Tokyo 104-8386 (JP)
(72) Inventor: KITA, Kiyoshi, Tokyo 113-8654 (JP); SUWA, Akiyuki, Kawachinagano-shi Osaka 586-0094 (JP); ODA, Masatsugu, Kawachinagano-shi Osaka 586-0094 (JP)
(74) Representative: Witte, Weller & Partner Patentanwälte mbB
(86) International application number: PCT/JP2015/056327
(87) International publication number: WO 2015/133512

(56) References cited:
- EP-A1- 1 997 800
- EP-A1- 2 682 115
- EP-A1- 2 891 492
- WO-A1-2007/108483
- WO-A1-2012/118139
- WO-A1-2014/034750

## Description

### TECHNICAL FIELD

The present invention relates an endoparasite control agent comprising a heterocyclic carboxamide derivative or a salt thereof as an active ingredient, and a method for controlling endoparasites, comprising orally or parenterally administering the endoparasite control agent.

### BACKGROUND ART

Certain kinds of carboxamide derivatives have been known to have microbicidal activity (see Patent Literature 1 to 14). However, the literature does not describe that these compounds are effective for the disinfection or control of endoparasites in animals such as mammals and birds. It is also known that certain kinds of carboxamide derivatives are effective against nematodes that may damage agricultural products (see Patent Literature 4 or 5), but there is no specific disclosure as to whether these compounds are effective against endoparasites in animals. Furthermore, there is a report that compounds that inhibit succinate-ubiquinone reductase (mitochondrial complex II), which is one of the respiratory enzymes of endoparasites, can serve as an endoparasite control agent (see Patent Literature 13 and Non Patent Literature 1). However, there is no disclosure of the compounds of the present invention.

Generally, parasitosis is caused by infestation of host animals with parasites such as unicellular protists (protozoa), multicellular helminths and arthropods. It is reported that the incidence of parasitosis in Japan has been remarkably decreased by improvement of environmental hygiene, but on a global scale, particularly in developing countries, parasitosis still widely prevails and causes tremendous damage. In recent years, there have been seen the introduction of infection sources via long- or short-term travelers having visited such countries; parasitic infection due to the consumption of food imports or raw meat and fish meat, which have become more available thanks to the advance in freezing and logistics technologies; and the transmission of parasitosis from pets. Under such circumstances, the incidence of parasitosis is on an upward trend again. Another problem is that immunodeficiency caused by mass administration of immunosuppressants, anticancer drugs, etc. or by AIDS etc. allows usually non-pathogenic or low-pathogenic parasites to express their pathogenicity and to cause opportunistic infection in hosts. Further, parasitosis in domestic animals, such as pigs, horses, cattle, sheep, dogs, cats and domestic fowls, is a universal and serious economic problem. That is, parasitic infection of domestic animals causes anemia, malnutrition, debility, weight loss, and serious damage of intestinal tract walls, tissues and organs, and may result in decline in feed efficiency and productivity, leading to a great economic loss. Therefore, novel parasiticides, antiprotozoals or other endoparasite control agents have always been desired.

### CITATION LIST

### Patent Literature

Patent Literature 1: JP-A 01-151546
Patent Literature 2: WO 2007/060162
Patent Literature 3: JP-A 53-9739
Patent Literature 4: WO 2007/108483 = EP 1 997 800
Patent Literature 5: WO 2007/104496
Patent Literature 6: WO 2008/101975
Patent Literature 7: WO 2008/101976
Patent Literature 8: WO 2008/003745
Patent Literature 9: WO 2008/003746
Patent Literature 10: WO 2009/012998
Patent Literature 11: WO 2009/127718
Patent Literature 12: WO 2010/106971
Patent Literature 13: WO 2012/118139 = EP 2 682 115
Patent Literature 14: WO 2014/034750 = EP 2 891 492

### Non Patent Literature

Non Patent Literature 1: Kiyoshi Kita, "Kansen (Infection)", Winter 2010, Vol. 40-4, 310-319

### SUMMARY OF INVENTION

### TECHNICAL PROBLEM

In view of the above-described circumstances, the present invention is mainly intended to provide a novel parasiticide, antiprotozoal or other endoparasite control agents which are effective for controlling animal endoparasites that have been impossible to control by conventional ones.

### SOLUTION TO PROBLEM

The present inventors conducted extensive research to solve the above-described problems. As a result, the present inventors found that a heterocyclic carboxamide derivative represented by the general formula (I) of the present invention and a salt thereof are highly effective for controlling endoparasites. The present inventors further conducted a great deal of examination and then completed the present invention. That is, the present invention relates to the following.
[1] An agent for use in the control of endoparasite in animals, said agent comprising, as an active ingredient, a heterocyclic carboxamide derivative represented by the general formula (I): 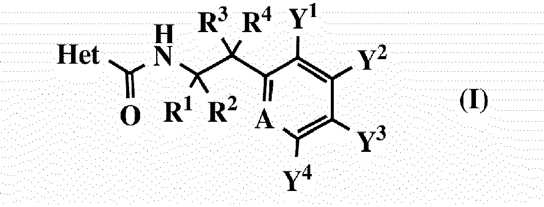 {wherein
   R¹ and R² may be the same or different, and are selected from the group consisting of
      (a1) a hydrogen atom;
      (a2) a (C₁-C₆) alkyl group; and
      (a3) a (C1-C6) alkoxy group, or optionally
      (a4) R¹ and R² together with the carbon atom bound to R¹ and R² form a (C₃-C₆) cycloalkane,
   R³ and R⁴ may be the same or different, and are selected from the group consisting of
      (b1) a hydrogen atom;
      (b2) a (C₁-C₆) alkyl group; and
      (b3) a (C₁-C₆) alkoxy group, or optionally
      (b4) R³ and R⁴ together with the carbon atom bound to R³ and R⁴ form a (C₃-C₆) cycloalkane,
   Y¹ is
      (c1) a hydrogen atom;
      (c2) a halogen atom;
      (c3) a cyano group;
      (c4) a nitro group;
      (c5) a (C₁-C₆) alkyl group;
      (c6) a (C₁-C₆) alkoxy group;
      (c7) a halo (C₁-C₆) alkyl group; or
      (c8) a halo (C₁-C₆) alkoxy group,
   Y² and Y⁴ may be the same or different, and are selected from the group consisting of
      (d1) a hydrogen atom;
      (d2) a halogen atom;
      (d3) a (C₁-C₆) alkyl group;
      (d4) a (C₁-C₆) alkoxy group;
      (d5) a halo (C₁-C₆) alkyl group; and
      (d6) a halo (C₁-C₆) alkoxy group,
   Y³ is selected from the group consisting of
      (e1) a phenyl group;
      (e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
      (e3) a phenoxy group;
      (e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e5) a pyridyl group;
      (e6) a pyridyl group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e7) a pyridyloxy group;
      (e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
      (e9) a pyrimidyloxy group;
      (e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e11) a pyrazyloxy group;
      (e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e13) a pyrazolyloxy group;
      (e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
      (e15) a quinolyloxy group;
      (e16) a quinolyloxy group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e17) a quinoxalyloxy group;
      (e18) a quinoxalyloxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e19) a benzoxazolyloxy group;
      (e20) a benzoxazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e21) a benzothiazolyloxy group;
      (e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e23) a furanyl group;
      (e24) a furanyl group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e25) a thienyl group;
      (e26) a thienyl group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e27) a naphthyl group;
      (e28) a naphthyl group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e29) a naphthyloxy group; and
      (e30) a naphthyloxy group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group,
   A is a nitrogen atom or a CZ group (wherein Z represents a hydrogen atom, (a) a halogen atom, (d) a (C₁-C₆) alkyl group, (e) a halo (C₁-C₆) alkyl group, (f) a (C₁-C₆) alkoxy group or (g) a halo (C₁-C₆) alkoxy group), and
   Het represents 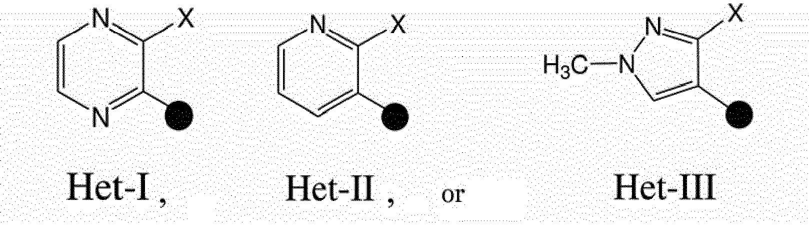 (wherein X represents a halogen atom or a halo (C₁-C₆) alkyl group, and the black circle represents a binding site)}, or a salt thereof.
[2] The agent for use according to the above [1], wherein
   R¹ and R² are (a1) hydrogen atoms,
   R³ and R⁴ may be the same or different, and are selected from the group consisting of
      (b1) a hydrogen atom;
      (b2) a (C₁-C₆) alkyl group; and
      (b3) a (C₁-C₆) alkoxy group, or optionally
      (b4) R³ and R⁴ together with the carbon atom bound to R³ and R⁴ form a (C₃-C₆) cycloalkane,
   Y¹ is (c1) a halogen atom,
   Y² and Y⁴ are (d1) hydrogen atoms, and
   Y³ is selected from the group consisting of
      (e1) a phenyl group;
      (e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
      (e3) a phenoxy group;
      (e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e5) a pyridyl group;
      (e6) a pyridyl group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e7) a pyridyloxy group;
      (e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
      (e9) a pyrimidyloxy group;
      (e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e11) a pyrazyloxy group;
      (e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e13) a pyrazolyloxy group;
      (e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
      (e15) a quinolyloxy group;
      (e16) a quinolyloxy group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e17) a quinoxalyloxy group;
      (e18) a quinoxalyloxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e19) a benzoxazolyloxy group;
      (e20) a benzoxazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e21) a benzothiazolyloxy group; and
      (e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group.
[3] The agent for use according to the above [1], wherein
   R¹ and R² are (a1) hydrogen atoms,
   R³ and R⁴ may be the same or different, and are selected from the group consisting of
      (b1) a hydrogen atom;
      (b2) a (C₁-C₆) alkyl group; and
      (b3) a (C₁-C₆) alkoxy group, or optionally
      (b4) R³ and R⁴ together with the carbon atom bound to R³ and R⁴ form a (C₃-C₆) cycloalkane,
   Y¹ is (c1) a halogen atom,
   Y² and Y⁴ are (d1) hydrogen atoms,
   Y³ is selected from the group consisting of
      (e1) a phenyl group;
      (e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
      (e3) a phenoxy group;
      (e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e5) a pyridyl group;
      (e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
      (e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e11) a pyrazyloxy group;
      (e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
      (e15) a quinolyloxy group;
      (e17) a quinoxalyloxy group;
      (e19) a benzoxazolyloxy group;
      (e21) a benzothiazolyloxy group; and
      (e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group, and
   Het is Het-I.
[4] A pyrazine carboxamide derivative represented by the general formula (I): 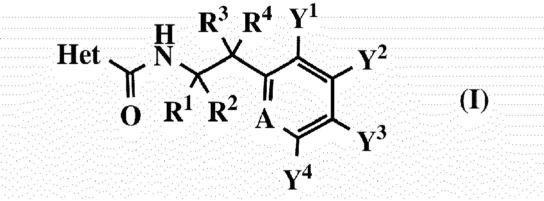 {wherein
   R¹ and R² are (a1) hydrogen atoms,
   R³ and R⁴ may be the same or different, and are selected from the group consisting of
      (b1) a hydrogen atom;
      (b2) a (C₁-C₆) alkyl group; and
      (b3) a (C₁-C₆) alkoxy group, or optionally
      (b4) R³ and R⁴ together with the carbon atom bound to R³ and R⁴ form a (C₃-C₆) cycloalkane,
   Y¹ is (c1) a halogen atom,
   Y² and Y⁴ are (d1) hydrogen atoms,
   Y³ is selected from the group consisting of
      (e1) a phenyl group;
      (e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
      (e3) a phenoxy group;
      (e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e5) a pyridyl group;
      (e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
      (e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e11) a pyrazyloxy group;
      (e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
      (e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
      (e15) a quinolyloxy group;
      (e17) a quinoxalyloxy group;
      (e19) a benzoxazolyloxy group;
      (e21) a benzothiazolyloxy group; and
      (e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group, and
   Het represents the following formula Het-I: 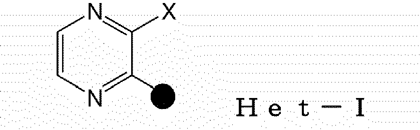 (wherein X represents a halogen atom or a halo (C₁-C₆) alkyl group, and the black circle represents a binding site)}, or a salt thereof.
[9] The heterocyclic carboxamide derivative specified in any one of the above [1] to [3] or a salt thereof for use in the control of endoparasites.

### ADVANTAGEOUS EFFECTS OF INVENTION

The present invention provides an endoparasite control agent having better performance in the disinfection or control of endoparasites as compared with the conventional art.

### DESCRIPTION OF EMBODIMENTS

The definitions in connection with the general formula (I) representing the heterocyclic carboxamide derivative of the present invention are described below.

The "halogen atom" refers to a chlorine atom, a bromine atom, an iodine atom or a fluorine atom.

The "(C₁-C₆) alkyl group" refers to a straight-chain or branched-chain alkyl group of 1 to 6 carbon atoms, for example, a methyl group, an ethyl group, a n-propyl group, an isopropyl group, a n-butyl group, an isobutyl group, a sec-butyl group, a tert-butyl group, a n-pentyl group, a neopentyl group, a n-hexyl group or the like.

The "(C₁-C₆) alkoxy group" refers to a straight-chain or branched-chain alkoxy group of 1 to 6 carbon atoms, for example, a methoxy group, an ethoxy group, a n-propoxy group, an isopropoxy group, a n-butoxy group, a sec-butoxy group, a tert-butoxy group, a n-pentyloxy group, an isopentyloxy group, a neopentyloxy group, a n-hexyloxy group or the like.

The "halo (C₁-C₆) alkyl group" refers to a straight-chain or branched-chain alkyl group of 1 to 6 carbon atoms substituted with one or more halogen atoms which may be the same or different from each other, for example, a trifluoromethyl group, a difluoromethyl group, a perfluoroethyl group, a hexafluoroisopropyl group, a perfluoroisopropyl group, a chloromethyl group, a bromomethyl group, a 1-bromoethyl group, a 2,3-dibromopropyl group or the like.

The "halo (C₁-C₆) alkoxy group" refers to a straight-chain or branched-chain alkoxy group of 1 to 6 carbon atoms substituted with one or more halogen atoms which may be the same or different from each other, for example, a trifluoromethoxy group, a difluoromethoxy group, a perfluoroethoxy group, a perfluoroisopropoxy group, a chloromethoxy group, a bromomethoxy group, a 1-bromoethoxy group, a 2,3-dibromopropoxy group or the like.

The "(C₃-C₆) cycloalkane" formed of R¹ and R² together with the carbon atom bound to R¹ and R² is, for example, cyclopropane, cyclobutane, cyclopentane, cyclohexane or the like.

Examples of the salt of the heterocyclic carboxamide derivative represented by the general formula (I) of the present invention include inorganic acid salts, such as hydrochlorides, sulfates, nitrates and phosphates; organic acid salts, such as acetates, fumarates, maleates, oxalates, methanesulfonates, benzenesulfonates and p-toluenesulfonates; and salts with an inorganic or organic base such as a sodium ion, a potassium ion, a calcium ion and a trimethylammonium ion.

As the heterocyclic carboxamide derivative of the present invention, preferred is a compound of the general formula (I) in which
R¹ and R² are (a1) hydrogen atoms,
R³ and R⁴ may be the same or different, and are selected from the group consisting of
   (b1) a hydrogen atom;
   (b2) a (C₁-C₆) alkyl group; and
   (b3) a (C₁-C₆) alkoxy group, or optionally
   (b4) R³ and R⁴ together with the carbon atom bound to R³ and R⁴ form a (C₃-C₆) cycloalkane,
Y¹ is (c1) a halogen atom,
Y² and Y⁴ are (d1) hydrogen atoms,
Y³ is selected from the group consisting of
   (e1) a phenyl group;
   (e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
   (e3) a phenoxy group;
   (e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e5) a pyridyl group;
   (e6) a pyridyl group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e7) a pyridyloxy group;
   (e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
   (e9) a pyrimidyloxy group;
   (e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e11) a pyrazyloxy group;
   (e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e13) a pyrazolyloxy group;
   (e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
   (e15) a quinolyloxy group;
   (e16) a quinolyloxy group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e17) a quinoxalyloxy group;
   (e18) a quinoxalyloxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e19) a benzoxazolyloxy group;
   (e20) a benzoxazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e21) a benzothiazolyloxy group; and
   (e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group, and
Het is Het-I, or
a salt thereof.

As the heterocyclic carboxamide derivative of the present invention, further preferred is a compound of the general formula (I) in which
R¹ and R² are (a1) hydrogen atoms,
R³ and R⁴ may be the same or different, and are selected from the group consisting of
   (b1) a hydrogen atom;
   (b2) a (C₁-C₆) alkyl group; and
   (b3) a (C₁-C₆) alkoxy group, or optionally
   (b4) R³ and R⁴ together with the carbon atom bound to R³ and R⁴ form a (C₃-C₆) cycloalkane,
Y¹ is (c1) a halogen atom,
Y² and Y⁴ are (d1) hydrogen atoms,
Y³ is selected from the group consisting of
   (e1) a phenyl group;
   (e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
   (e3) a phenoxy group;
   (e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e5) a pyridyl group;
   (e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
   (e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e11) a pyrazyloxy group;
   (e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
   (e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
   (e15) a quinolyloxy group;
   (e17) a quinoxalyloxy group;
   (e19) a benzoxazolyloxy group;
   (e21) a benzothiazolyloxy group; and
   (e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group, and
Het is Het-I, or
a salt thereof.

The compound represented by the general formula (I) of the present invention can be produced by the method described in JP-A 01-151546, WO 2007/060162, JP-A 53-9739, WO 2007/108483, WO 2008/101975, WO 2008/101976, WO 2008/003745, WO 2008/003746, WO 2009/012998, WO 2009/127718 or WO 2010/106971, the method described in WO 2012/118139 or Shin-Jikken Kagaku Kouza 14 (Maruzen, December 20, 1977), a modified method of the foregoing, or the like.

Representative examples of the heterocyclic carboxamide derivative represented by the general formula (I) of the present invention are shown in Tables 1, 2 and 3, but the present invention is not limited thereto. In Tables 1, 2 and 3, "Me" stands for a methyl group, "Ph" stands for a phenyl group, "Py" stands for a pyridyl group, "PhO" stands for a phenoxy group, and "PyO" stands for a pyridyloxy group. Shown in the column of "Physical property" are mass spectral data.

Q1 to Q19 represent the following structures. The black circle in each of the formulae Q1 to Q19 represents a binding site.

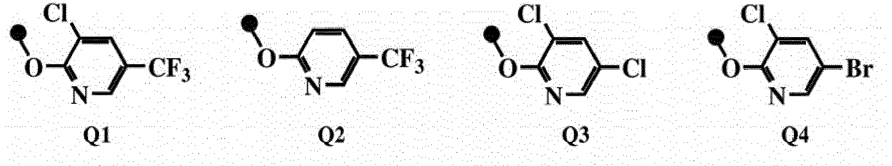

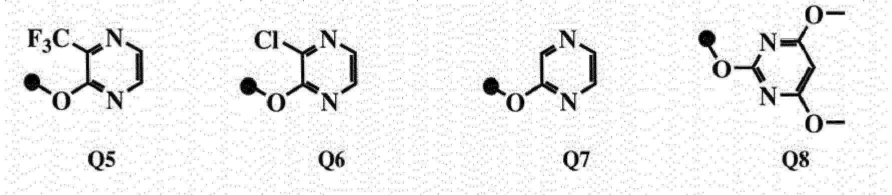

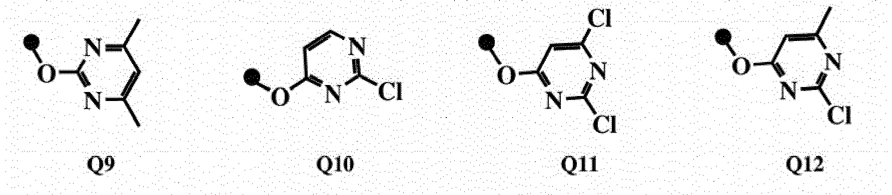

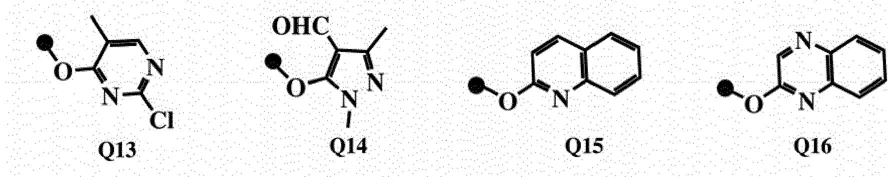

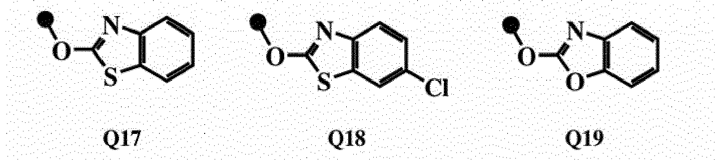

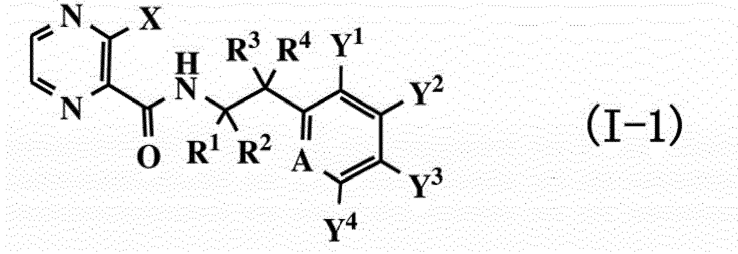

**Table 1**

| Compound No. | X | R³ | R⁴ | Y³ | A | Physical property |
|---|---|---|---|---|---|---|
| 1-1 | CF₃ | H | H | Q1 | CH | 535 (M+1) |
| 1-2 | CF₃ | H | H | Q2 | CH | 491 (M+1) |
| 1-3 | CF₃ | H | H | Q3 | CH | 491 (M+1) |
| 1-4 | CF₃ | H | H | Q4 | CH | 537 (M+1) |
| 1-5 | CF₃ | H | H | Q5 | CH | 592 (M+1) |
| 1-6 | CF₃ | H | H | Q6 | CH | 558 (M+1) |
| 1-7 | CF₃ | H | H | Q7 | CH | 424 (M+1) |
| 1-8 | CF₃ | H | H | Q8 | CH | 484 (M+1) |
| 1-9 | CF₃ | H | H | Q9 | CH | 452 (M+1) |
| 1-10 | CF₃ | H | H | Q10 | CH | 458 (M+1) |
| 1-11 | CF₃ | H | H | Q11 | CH | 492 (M+1) |
| 1-12 | CF₃ | H | H | Q12 | CH | 472 (M+1) |
| 1-13 | CF₃ | H | H | Q13 | CH | 472 (M+1) |
| 1-14 | CF₃ | H | H | Q14 | CH | 468 (M+1) |
| 1-15 | CF₃ | H | H | Q15 | CH | 473 (M+1) |
| 1-16 | CF₃ | H | H | Q16 | CH | 474 (M+1) |
| 1-17 | CF₃ | H | H | Q17 | CH | 479 (M+1) |
| 1-18 | CF₃ | H | H | Q18 | CH | 513 (M+1) |
| 1-19 | CF₃ | H | H | Q19 | CH | 463 (M+1) |
| 1-20 | CF₃ | H | H | PhO | CH | 422 (M+1) |
| 1-21 | CF₃ | H | H | 4-CF₃-PhO | CH | 490 (M+1) |
| 1-22 | CF₃ | Me | H | Q1 | CH | 539 (M+1) |
| 1-23 | CF₃ | Me | H | Q2 | CH | 505 (M+1) |
| 1-24 | CF₃ | Me | H | Q3 | CH | 505 (M+1) |
| 1-25 | CF₃ | Me | Me | Q1 | CH | 553 (M+1) |
| 1-26 | CF₃ | Me | Me | Q2 | CH | 519 (M+1) |
| 1-27 | CF₃ | Me | Me | Q3 | CH | 519 (M+1) |
| 1-28 | CF₃ | CH₂CH₂ | | Q1 | CH | 551 (M+1) |
| 1-29 | CF₃ | CH₂CH₂ | | Q2 | CH | 517 (M+1) |
| 1-30 | CF₃ | CH₂CH₂ | | Q3 | CH | 517 (M+1) |
| 1-31 | CF₃ | H | H | 4-CF₃-Ph | CH | 474 (M+1) |
| 1-32 | CF₃ | H | H | 3-CF₃-Ph | CH | 474 (M+1) |
| 1-33 | CF₃ | H | H | 3,5-F₂-Ph | CH | 442 (M+1) |
| 1-34 | CF₃ | H | H | 4-MeO-PhO | CH | 436 (M+1) |
| 1-35 | CF₃ | H | H | Ph | CH | 406 (M+1) |
| 1-36 | CF₃ | H | H | 4-Py | CH | 407 (M+1) |
| 1-37 | CF₃ | H | H | 3-Py | CH | 407 (M+1) |
| 1-38 | CF₃ | MeO | H | Q1 | CH | 555 (M+1) |
| 1-39 | CF₃ | MeO | H | Q2 | CH | 521 (M+1) |
| 1-40 | CF₃ | MeO | H | Q3 | CH | 523 (M+1) |
| 1-41 | CF₃ | MeO | H | Q17 | CH | 509 (M+1) |
| 1-42 | CF₃ | MeO | H | Ph | CH | 420 (-MeOH) |
| 1-43 | CF₃ | MeO | H | 4-CF₃-Ph | CH | 488 (-MeOH) |
| 1-44 | CF₃ | MeO | H | 4-MeO-PhO | CH | 450 (-MeOH) |
| 1-45 | CF₃ | MeO | H | 3-PyO | CH | 453 (-MeOH) |
| 1-46 | CF₃ | H | H | Ph | N | |
| 1-47 | CF₃ | H | H | 4-CF₃-Ph | N | |
| 1-48 | CF₃ | H | H | 3-CF₃-Ph | N | |
| 1-49 | CF₃ | H | H | 3,5-F₂-Ph | N | |
| 1-50 | CF₃ | Me | H | Ph | N | 421 (M+1) |
| 1-51 | CF₃ | Me | H | 4-CF₃-Ph | N | |
| 1-52 | CF₃ | Me | H | 3-CF₃-Ph | N | |
| 1-53 | CF₃ | Me | H | 3,5-F₂-Ph | N | |
| 1-54 | CF₃ | Me | Me | Ph | N | |
| 1-55 | CF₃ | Me | Me | 4-CF₃-Ph | N | |
| 1-56 | CF₃ | Me | Me | 3-CF₃-Ph | N | |
| 1-57 | CF₃ | Me | Me | 3,5-F₂-Ph | N | |
| In Table 1, R¹ and R² represent H, Y¹ represents Cl, and Y² and Y⁴ represent H. | | | | | | |

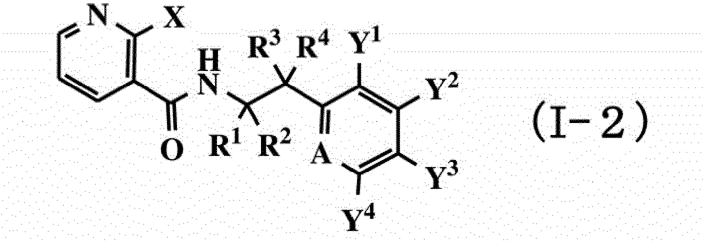

**Table 2**

| Compound No. | X | R³ | R⁴ | Y³ | A | Physical property |
|---|---|---|---|---|---|---|
| 2-1 | CF₃ | H | H | Q1 | CH | |
| 2-2 | CF₃ | H | H | Q2 | CH | |
| 2-3 | CF₃ | H | H | Q3 | CH | |
| 2-4 | CF₃ | H | H | Q4 | CH | |
| 2-5 | CF₃ | H | H | Q1 | CH | |
| 2-6 | CF₃ | H | H | Q2 | CH | |
| 2-7 | CF₃ | H | H | Q3 | CH | |
| 2-8 | CF₃ | H | H | Q4 | CH | |
| 2-9 | CF₃ | H | H | Q1 | CH | |
| 2-10 | CF₃ | H | H | Q2 | CH | |
| 2-11 | CF₃ | H | H | Q3 | CH | |
| 2-12 | CF₃ | H | H | Q4 | CH | |
| 2-13 | CF₃ | H | H | Q1 | CH | |
| 2-14 | CF₃ | H | H | Q2 | CH | |
| 2-15 | CF₃ | H | H | Q3 | CH | |
| 2-16 | CF₃ | H | H | Q4 | CH | |
| 2-17 | CF₃ | H | H | Q1 | CH | |
| 2-18 | CF₃ | H | H | Q2 | CH | |
| 2-19 | CF₃ | H | H | Q3 | CH | |
| 2-20 | CF₃ | H | H | PhO | CH | |
| 2-21 | CF₃ | H | H | 4-CF₃-PhO | CH | |
| 2-22 | CF₃ | Me | H | Q1 | CH | |
| 2-23 | CF₃ | Me | H | Q2 | CH | |
| 2-24 | CF₃ | Me | H | Q3 | CH | |
| 2-25 | CF₃ | Me | Me | Q1 | CH | |
| 2-26 | CF₃ | Me | Me | Q2 | CH | |
| 2-27 | CF₃ | Me | Me | Q3 | CH | |
| 2-28 | CF₃ | CH₂CH₂ | | Q1 | CH | |
| 2-29 | CF₃ | CH₂CH₂ | | Q2 | CH | |
| 2-30 | CF₃ | CH₂CH₂ | | Q3 | CH | |
| 2-31 | CF₃ | H | H | 4-CF₃-Ph | CH | |
| 2-32 | CF₃ | H | H | 3-CF₃-Ph | CH | |
| 2-33 | CF₃ | H | H | 3,5-F₂-Ph | CH | |
| 2-34 | CF₃ | H | H | 4-MeO-PhO | CH | |
| 2-35 | CF₃ | H | H | Ph | CH | |
| 2-36 | CF₃ | H | H | 4-Py | CH | |
| 2-37 | CF₃ | H | H | 3-Py | CH | |
| 2-38 | CF₃ | MeO | H | Q1 | CH | |
| 2-39 | CF₃ | MeO | H | Q2 | CH | |
| 2-40 | CF₃ | MeO | H | Q3 | CH | |
| 2-41 | CF₃ | MeO | H | Q17 | CH | |
| 2-42 | CF₃ | MeO | H | PhO | CH | |
| 2-43 | CF₃ | MeO | H | 4-CF₃-PhO | CH | |
| 2-44 | CF₃ | MeO | H | 4-MeO-PhO | CH | |
| 2-45 | CF₃ | MeO | H | 3-PyO | CH | |
| 2-46 | Cl | H | H | Ph | N | |
| 2-47 | Cl | H | H | 4-CF₃-Ph | N | |
| 2-48 | Cl | H | H | 3-CF₃-Ph | N | |
| 2-49 | Cl | H | H | 3,5-F₂-Ph | N | |
| 2-50 | Cl | Me | H | Ph | N | |
| 2-51 | Cl | Me | H | 4-CF₃-Ph | N | |
| 2-52 | Cl | Me | H | 3-CF₃-Ph | N | |
| 2-53 | Cl | Me | H | 3,5-F₂-Ph | N | |
| 2-54 | Cl | Me | Me | Ph | N | |
| 2-55 | Cl | Me | Me | 4-CF₃-Ph | N | |
| 2-56 | Cl | Me | Me | 3-CF₃-Ph | N | |
| 2-57 | Cl | Me | Me | 3,5-F₂-Ph | N | |
| In Table 2, R¹ and R² represent H, Y¹ represents Cl, and Y² and Y⁴ represent H. | | | | | | |

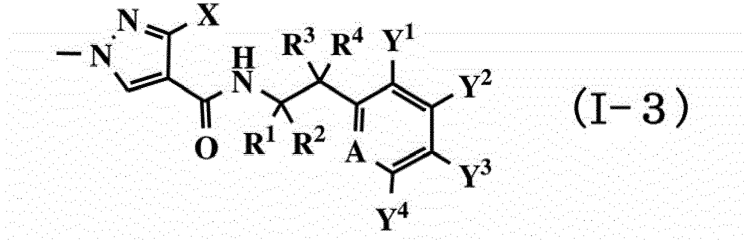

**Table 3**

| Compound No. | X | R³ | R⁴ | Y³ | A | Physical property |
|---|---|---|---|---|---|---|
| 3-1 | CF₃ | H | H | Q1 | CH | |
| 3-2 | CF₃ | H | H | Q2 | CH | |
| 3-3 | CF₃ | H | H | Q3 | CH | |
| 3-4 | CF₃ | H | H | Q4 | CH | |
| 3-5 | CF₃ | H | H | Q5 | CH | |
| 3-6 | CF₃ | H | H | Q6 | CH | |
| 3-7 | CF₃ | H | H | Q7 | CH | |
| 3-8 | CF₃ | H | H | Q8 | CH | |
| 3-9 | CF₃ | H | H | Q9 | CH | |
| 3-10 | CF₃ | H | H | Q10 | CH | |
| 3-11 | CF₃ | H | H | Q11 | CH | |
| 3-12 | CF₃ | H | H | Q12 | CH | |
| 3-13 | CF₃ | H | H | Q13 | CH | |
| 3-14 | CF₃ | H | H | Q14 | CH | |
| 3-15 | CF₃ | H | H | Q15 | CH | |
| 3-16 | CF₃ | H | H | Q16 | CH | |
| 3-17 | CF₃ | H | H | Q17 | CH | |
| 3-18 | CF₃ | H | H | Q18 | CH | |
| 3-19 | CF₃ | H | H | Q19 | CH | |
| 3-20 | CF₃ | H | H | PhO | CH | |
| 3-21 | CF₃ | H | H | 4-CF₃-Ph | CH | |
| 3-22 | CF₃ | Me | H | Q1 | CH | |
| 3-23 | CF₃ | Me | H | Q2 | CH | |
| 3-24 | CF₃ | Me | H | Q3 | CH | |
| 3-25 | CF₃ | Me | Me | Q1 | CH | |
| 3-26 | CF₃ | Me | Me | Q2 | CH | |
| 3-27 | CF₃ | Me | Me | Q3 | CH | |
| 3-28 | CF₃ | CH₂CH₂ | | Q1 | CH | |
| 3-29 | CF₃ | CH₂CH₂ | | Q2 | CH | |
| 3-30 | CF₃ | CH₂CH₂ | | Q3 | CH | |
| 3-31 | CF₃ | H | H | 4-CF₃-Ph | CH | |
| 3-32 | CF₃ | H | H | 3-CF₃-Ph | CH | |
| 3-33 | CF₃ | H | H | 3,5-F₂-Ph | CH | |
| 3-34 | CF₃ | H | H | 4-MeO-PhO | CH | |
| 3-35 | CF₃ | H | H | Ph | CH | |
| 3-36 | CF₃ | H | H | 4-Py | CH | |
| 3-37 | CF₃ | H | H | 3-Py | CH | |
| 3-38 | CF₃ | MeO | H | Q1 | CH | |
| 3-39 | CF₃ | MeO | H | Q2 | CH | |
| 3-40 | CF₃ | MeO | H | Q3 | CH | |
| 3-41 | CF₃ | MeO | H | Q17 | CH | |
| 3-42 | CF₃ | MeO | H | PhO | CH | |
| 3-43 | CF₃ | MeO | H | 4-CF₃-PhO | CH | |
| 3-44 | CF₃ | MeO | H | 4-MeO-PhO | CH | |
| 3-45 | CF₃ | MeO | H | 3-PyO | CH | |
| 3-46 | CHF₂ | H | H | Q1 | CH | |
| 3-47 | CHF₂ | H | H | Q2 | CH | |
| 3-48 | CHF₂ | H | H | Q3 | CH | |
| 3-49 | CHF₂ | H | H | Q4 | CH | |
| 3-50 | CHF₂ | H | H | Q5 | CH | |
| 3-51 | CHF₂ | H | H | Q6 | CH | |
| 3-52 | CHF₂ | H | H | Q7 | CH | |
| 3-53 | CHF₂ | H | H | Q8 | CH | |
| 3-54 | CHF₂ | H | H | Q9 | CH | |
| 3-55 | CHF₂ | H | H | Q10 | CH | |
| 3-56 | CHF₂ | H | H | Q11 | CH | |
| 3-57 | CHF₂ | H | H | Q12 | CH | |
| 3-58 | CHF₂ | H | H | Q13 | CH | |
| 3-59 | CHF₂ | H | H | Q14 | CH | |
| 3-60 | CHF₂ | H | H | Q15 | CH | |
| 3-61 | CHF₂ | H | H | Q16 | CH | |
| 3-62 | CHF₂ | H | H | Q17 | CH | |
| 3-63 | CHF₂ | H | H | Q18 | CH | |
| 3-64 | CHF₂ | H | H | Q19 | CH | |
| 3-65 | CHF₂ | H | H | PhO | CH | |
| 3-66 | CHF₂ | H | H | 4-CF₃-PhO | CH | |
| 3-67 | CHF₂ | Me | H | Q1 | CH | |
| 3-68 | CHF₂ | Me | H | Q2 | CH | |
| 3-69 | CHF₂ | Me | H | Q3 | CH | |
| 3-70 | CHF₂ | Me | Me | Q1 | CH | |
| 3-71 | CHF₂ | Me | Me | Q2 | CH | |
| 3-72 | CHF₂ | Me | Me | Q3 | CH | |
| 3-73 | CHF₂ | CH₂CH₂ | | Q1 | CH | |
| 3-74 | CHF₂ | CH₂CH₂ | | Q2 | CH | |
| 3-75 | CHF₂ | CH₂CH₂ | Q3 | CH | | |
| 3-76 | CHF₂ | H | H | 4-CF₃-Ph | CH | |
| 3-77 | CHF₂ | H | H | 3-CF₃-Ph | CH | |
| 3-78 | CHF₂ | H | H | 3,5-F₂-Ph | CH | |
| 3-79 | CHF₂ | H | H | 4-MeO-PhO | CH | |
| 3-80 | CHF₂ | H | H | Ph | CH | |
| 3-81 | CHF₂ | H | H | 4-Py | CH | |
| 3-82 | CHF₂ | H | H | 3-Py | CH | |
| 3-83 | CHF₂ | MeO | H | Q1 | CH | |
| 3-84 | CHF₂ | MeO | H | Q2 | CH | |
| 3-85 | CHF₂ | MeO | H | Q3 | CH | |
| 3-86 | CHF₂ | MeO | H | Q17 | CH | |
| 3-87 | CHF₂ | MeO | H | PhO | CH | |
| 3-88 | CHF₂ | MeO | H | 4-CF₃-PhO | CH | |
| 3-89 | CHF₂ | MeO | H | 4-MeO-PhO | CH | |
| 3-90 | CHF₂ | MeO | H | 3-PyO | CH | |
| 3-91 | CF₃ | H | H | Ph | N | |
| 3-92 | CF₃ | H | H | 4-CF₃-Ph | N | |
| 3-93 | CF₃ | H | H | 3-CF₃-Ph | N | |
| 3-94 | CF₃ | H | H | 3,5-F₂-Ph | N | |
| 3-95 | CF₃ | Me | H | Ph | N | |
| 3-96 | CF₃ | Me | H | 4-CF₃Ph | N | |
| 3-97 | CF₃ | Me | H | 3-CF₃-Ph | N | |
| 3-98 | CF₃ | Me | H | 3,5-F₂-Ph | N | |
| 3-99 | CF₃ | Me | Me | Ph | N | |
| 3-100 | CF₃ | Me | Me | 4-CF₃-Ph | N | |
| 3-101 | CF₃ | Me | Me | 3-CF₃-Ph | N | |
| 3-102 | CF₃ | Me | Me | 3,5-F₂-Ph | N | |
| 3-103 | CHF₂ | H | H | Ph | N | |
| 3-104 | CHF₂ | H | H | 4-CF₃-Ph | N | |
| 3-105 | CHF₂ | H | H | 3-CF₃-Ph | N | |
| 3-106 | CHF₂ | H | H | 3,5-F₂-Ph | N | |
| 3-107 | CHF₂ | Me | H | Ph | N | |
| 3-108 | CHF₂ | Me | H | 4-CF₃-Ph | N | |
| 3-109 | CHF₂ | Me | H | 3-CF₃-Ph | N | |
| 3-110 | CHF₂ | Me | H | 3,5-F₂-Ph | N | |
| 3-111 | CHF₂ | Me | Me | Ph | N | |
| 3-112 | CHF₂ | Me | Me | 4-CF₃-Ph | N | |
| 3-113 | CHF₂ | Me | Me | 3-CF₃-Ph | N | |
| 3-114 | CHF₂ | Me | Me | 3,5-F₂-Ph | N | |

| | | | | | | |
|---|---|---|---|---|---|---|
| In Table 3, R¹ and R² represent H, Y¹ represents Cl, and Y² and Y⁴ represent H. | | | | | | |

The endoparasite control agent of the present invention has excellent anti-endoparasite effect, and exerts appropriate control effect against endoparasites. The animal for which the endoparasite control agent of the present invention can be used is a human and an animal of non-human mammalian or avian species . Exemplary members of the non-human mammalian species include domestic animals, such as pigs, horses, cattle, sheep, goats, rabbits, camels, water buffalos, deer, mink and chinchillas; pet animals, such as dogs, cats, little birds and monkeys; and experimental animals, such as rats, mice, golden hamsters and guinea pigs. Exemplary members of the avian species include domestic fowls, such as chickens, ducks, aigamo ducks (crossbreeds of wild and domestic ducks), quails, domestic ducks, geese and turkeys. The examples listed above are non-limiting examples.

Human endoparasites against which the endoparasite control agent of the present invention is effective are roughly classified into protozoa and helminths. Examples of the protozoa include, but are not limited thereto, Rhizopoda, such as *Entamoeba histolytica;* Mastigophora, such as *Leishmania, Trypanosoma* and *Trichomonas;* Sporozoea, such as *Plasmodium* and *Toxoplasma;* and Ciliophora, such as *Balantidium coli.* Examples of the helminths include, but are not limited thereto, Nematoda, such as *Ascaris lumbricoides, Anisakis, Toxocara canis, Trichostrongylus* spp., *Enterobius vermicularis,* hookworms (for example, *Ancylostoma duodenale, Necator americanus, Ancylostoma braziliense,* etc.), *Angiostrongylus* spp., *Gnathostoma* spp., filarial worms (filaria, *Wuchereria bancrofti, Brugia malayi,* etc.), *Onchocerca volvulus, Dracunculus medinensis, Trichinella spiralis* and *Strongyloides stercoralis;* Acanthocephala, such as *Macracanthorhynchus hirudinaceus;* Gordiacea, such as Gordioidea; Hirudinea, such as *Hirudo nipponia;* Trematoda, such as *Schistosoma japonicum, Schistosoma mansoni, Schistosoma haematobium, Clonorchis sinensis, Heterophyes heterophyes, Fasciola* spp. and *Paragonimus* spp.; and Cestoda, such as *Diphyllobothrium latum, Sparganum mansoni, Sparganum proliferum, Diplogonoporus grandis,* Taeniidae (for example, *Taeniarhynchus saginatus, Taenia solium, Echinococcus,* etc.), *Hymenolepis* spp., *Dipylidium caninum, Mesocestoides lineatus, Bertiella* spp. and *Nybelinia surmenicola.*

Non-human mammalian or avian endoparasites against which the endoparasite control agent of the present invention is effective are roughly classified into protozoa and helminths. Examples of the protozoa include, but are not limited thereto, Apicomplexa, such as Coccidia (for example, *Eimeria, Isospora, Toxoplasma, Neospora, Sarcocystis, Besnoitia, Hammondia, Cryptosporidium, Caryospora,* etc.), Haemosporina (for example, *Leucocytozoon, Plasmodium,* etc.), Piroplasma (for example, *Theileria, Anaplasma, Eperythrozoon, Haemobartonella, Ehrlichia,* etc.), and others (for example, *Hepatozoon, Haemogregarina,* etc.); Microspora, such as *Encephalitozoon* and *Nosema;* Mastigophora, such as Trypanosomatid (for example, *Trypanosoma, Leishmania,* etc.), Trichomonadida (for example, *Chilomastix, Trichomonas, Monocercomonas, Histomonas,* etc.), and Diplomonadida (for example, *Hexamita, Giardia,* etc.); Sarcodina, such as Amoebida (for example, *Entamoeba histolytica (Entamoeba)* etc.); and Ciliophora, such as *Balantidium coli (Balantidium), Buxtonella* and *Entodinium.*

Examples of the helminths include, but are not limited thereto, Nematoda, such as Ascaridida (for example, *Ascaris suum (Ascaris), Toxocara canis* and *Toxocara cati (Toxocara), Toxascaris leonina (Toxascaris), Parascaris equorum (Parascaris), Ascaridia galli (Ascaridia), Heterakis gallinarum (Heterakis), Anisakis,* etc.), Oxyurida (for example, *Oxyuris equi (Oxyuris), Passalurus ambiguus (Passalurus),* etc.), Strongylida (for example, *Strongylus vulgaris (Strongylus), Haemonchus contortus (Haemonchus), Ostertagia ostertagi (Ostertagia), Trichostrongylus colubriformis* (*Trichostrongylus*)*, Cooperia punctata* (*Cooperia*)*, Nematodirus filicollis (Nematodirus), Hyostrongylus rubidus* (*Hyostrongylus*), *Oesophagostomum radiatum (Oesophagostomum), Chabertia ovina (Chabertia), Ancylostoma caninum (Ancylostoma), Uncinaria stenocephala (Uncinaria), Necator americanus (Necator), Bunostomum phlebotomum (Bunostomum), Dictyocaulus viviparus (Dictyocaulus), Metastrongylus elongatus (Metastrongylus), Filaroides hirthi (Filaroides), Aelurostrongylus abstrusus (Aelurostrongylus), Angiostrongylus cantonensis (Angiostrongylus), Syngamus trachea* (*Syngamus*), *Stephanurus dentatus (Stephanurus),* etc.), Rhabditida (for example, *Strongyloides stercoralis (Strongyloides), Micronema,* etc.), Spirurida (for example, *Thelazia rhodesi* (*Thelazia*)*, Oxyspirura mansoni* (*Oxyspirura*), *Spirocerca lupi (Spirocerca), Gongylonema pulchrum (Gongylonema), Draschia megastoma (Draschia), Habronema microstoma (Habronema), Ascarops strongylina (Ascarops), Physaloptera praeputialis (Physaloptera), Gnathostoma spinigerum (Gnathostoma),* etc.), Filariida (for example, *Dirofilaria immitis (Dirofilaria), Setaria equina (Setaria), Dipetalonema, Parafilaria multipapillosa (Parafilaria), Onchocerca cervicalis (Onchocerca),* etc.), and Enoplida (for example, *Parafilaria bovicola* (*Parafilaria*), *Stephanofilaria okinawaensis* (*Stephanofilaria*), *Trichuris vulpis* (*Trichuris*), *Capillaria bovis (Capillaria), Trichosomoides crassicauda (Trichosomoides), Trichinella spiralis (Trichinella), Dioctophyma renale (Dioctophyma),* etc.); Trematoda, such as Fasciolata (for example, *Fasciola hepatica (Fasciola), Fasciolopsis buski (Fasciolopsis),* etc.), Paramphistomatidae (for example, *Homalogaster paloniae (Homalogaster),* etc.), Dicrocoelata (for example, *Eurytrema pancreaticum* (*Eurytrema*), *Dicrocoelium dendriticum (Dicrocoelium),* etc.), Diplostomata (for example, *Pharyngostomum cordatum (Pharyngostomum), Alaria,* etc.), Echinostomata (for example, *Echinostoma hortense (Echinostoma), Echinochasmus,* etc.), Troglotrematoidea (for example, lung flukes *(Paragonimus), Nanophyetus salmincola (Nanophyetus),* etc.), Opisthorchiida (for example, *Clonorchis sinensis (Clonorchis)* etc.), Heterophyida (for example, *Heterophyes heterophyes (Heterophyes), Metagonimus yokogawai (Metagonimus),* etc.), Plagiorchiida (for example, *Prosthogonimus ovatus (Prosthogonimus)* etc.), and Schistosomatidae (for example, *Schistosoma japonicum (Schistosoma)* etc.); Cestoda, such as Pseudophylidea (for example, *Diphyllobothrium nihonkaiense (Diphyllobothrium), Spirometra erinacei (Spirometra),* etc.), and Cyclophyllidea (for example, *Anoplocephala perfoliata (Anoplocephala), Paranoplocephala mamillana (Paranoplocephala), Moniezia benedeni (Moniezia), Dipylidium caninum* (*Dipylidium*), *Mesocestoides lineatus* (*Mesocestoides*), *Taenia pisiformis* and *Taenia hydatigena* (*Taenia*), *Hydatigera taeniaeformis (Hydatigera), Multiceps multiceps (Multiceps), Echinococcus granulosus (Echinococcus), Echinococcus multilocularis (Echinococcus), Taenia solium (Taenia), Taeniarhynchus saginatus (Taeniarhynchus), Hymenolepis diminuta (Hymenolepis), Vampirolepis nana (Vampirolepis), Raillietina tetragona (Raillietina), Amoebotaenia sphenoides (Amoebotaenia),* etc.); Acanthocephala, such as *Macracanthorhynchus hirudinaceus (Macracanthorhynchus)* and *Moniliformis moniliformis (Moniliformis);* Linguatulida, such as *Linguatula serrata (Linguatula);* and other various parasites.

In different designations, examples of the helminths include, but are not limited to, Nematoda, such as Enoplida (for example, *Trichuris* spp., *Capillaria* spp., *Trichomosoides* spp. , *Trichinella* spp., etc.), Rhabditia (for example, *Micronema* spp., *Strongyloides* spp., etc.), Strongylida (for example, *Strongylus* spp., *Triodontophorus* spp., *Oesophagodontus* spp., *Trichonema* spp., *Gyalocephalus* spp., *Cylindropharynx* spp., *Poteriostomum* spp., *Cyclococercus* spp., *Cylicostephanus* spp., *Oesophagostomum* spp., *Chabertia* spp., *Stephanurus* spp., *Ancylostoma* spp., *Uncinaria* spp., *Bunostomum* spp., *Globocephalus* spp., *Syngamus* spp., *Cyathostoma* spp., *Metastrongylus* spp., *Dictyocaulus* spp., *Muellerius* spp., *Protostrongylus* spp., *Neostrongylus* spp., *Cystocaulus* spp., *Pneumostrongylus* spp., *Spicocaulus* spp., *Elaphostrongylus* spp., *Parelaphostrongylus* spp., *Crenosoma* spp., *Paracrenosoma* spp., *Angiostrongylus* spp., *Aelurostrongylus* spp., *Filaroides* spp., *Parafilaroides* spp., *Trichostrongylus* spp., *Haemonchus* spp., *Ostertagia* spp., *Marshallagia* spp., *Cooperia* spp., *Nematodirus* spp., *Hyostrongylus* spp., *Obeliscoides* spp., *Amidostomum* spp., *Ollulanus* spp., etc.),

Oxyurida (for example, *Oxyuris* spp., *Enterobius* spp., *Passalurus* spp., *Syphacia* spp., *Aspiculuris* spp., *Heterakis* spp., etc.), Ascaridia (for example, *Ascaris* spp., *Toxascaris* spp., *Toxocara* spp., *Parascaris* spp., *Anisakis* spp., *Ascaridia* spp., etc.), Spirurida (for example, *Gnathostoma* spp., *Physaloptera* spp., *Thelazia* spp., *Gongylonema* spp., *Habronema* spp., *Parabronema* spp., *Draschia* spp., *Dracunculus* spp., etc.), and Filariida (for example, *Stephanofilaria* spp., *Parafilaria* spp., *Setaria* spp., *Loa* spp., *Dirofilaria* spp., *Litomosoides* spp., *Brugia* spp., *Wuchereria* spp., *Onchocerca* spp., etc.);

Acanthocephala (for example, *Filicollis* spp., *Moniliformis* spp., *Macracanthorhynchus* spp., *Prosthenorchis* spp., etc.); Trematoda including subclasses, such as Monogenea (for example, *Gyrodactylus* spp., *Dactylogyrus* spp., *Polystoma* spp., etc.) and Digenea (for example, *Diplostomum* spp., *Posthodiplostomum* spp., *Schistosoma* spp., *Trichobilharzia* spp., *Ornithbilharzia* spp., *Austrobilharzia* spp., *Gigantobilharzia* spp., *Leucochloridium* spp., *Brachylaima* spp., *Echinostoma* spp., *Echinoparyphium* spp., *Echinochasmus* spp., *Hypoderaeum* spp., *Fasciola* spp., *Fasciolides* spp., *Fasciolopsis* spp., *Cyclocoelum* spp., *Typhlocoelum* spp., *Paramphistomum* spp., *Calicophoron* spp., *Cotylophoron* spp., *Gigantcotyle* spp., *Fischoederius* spp., *Gastrothylacus* spp., *Notocotylus* spp., *Catatropis* spp., *Plagiorchis* spp., *Prosthogonimus* spp., *Dicrocoelium* spp., *Eurytrema* spp., *Troglotrema* spp., *Paragonimus* spp., *Collyriclum* spp., *Nanophyetus* spp., *Opisthorchis* spp., *Clonorchis* spp., *Metorchis* spp., *Heterophyes* spp., *Metagonimus* spp., etc.);

Cestoda, such as Pseudophyllidea (for example, *Diphyllobothrium* spp., *Spirometra* spp., *Schistocephalus* spp., *Ligula* spp., *Bothridium* spp., *Diplogonoporus* spp., etc.), and Cyclophyllidea (for example, *Mesocestoides* spp., *Anoplocephala* spp., *Paranoplocehala* spp., *Moniezia* spp., *Thysanosomsa* spp., *Thysaniezia* spp., *Avitellina* spp., *Stilesia* spp., *Cittotaenia* spp., *Andyra* spp., *Bertiella* spp., *Taenia* spp., *Echinococcus* spp., *Hydatigera* spp., *Davainea* spp., *Raillietina* spp., *Hymenolepis* spp., *Echinolepis* spp., *Echinocotyle* spp., *Diorchis* spp., *Dipylidium* spp., *Joyeuxiella* spp., *Diplopylidium* spp., etc.); and others including parasites belonging to Acanthocephala and Linguatulida.

The endoparasite control agent of the present invention is effective for controlling not only parasites that live in the body of an intermediate or final host, but also parasites that live in the body of a reservoir host. The compound represented by the general formula (I) of the present invention is effective for controlling parasites at their every developmental stage. For example, in the case of protozoa, the compound is effective against their cysts, precystic forms and trophozoites; schizonts and amoeboid forms at the asexual stage; gametocytes, gametes and zygotes at the sexual stage; sporozoites; etc. In the case of nematodes, the compound is effective against their eggs, larvae and adults. The compound of the present invention is capable of not only combating parasites in the living body, but also even preventing parasitic infection by application to the environment as a route of infection. For example, soil-borne infection, i.e., infection from soil of crop fields and parks; percutaneous infection from water in rivers, lakes, marshes, paddy fields, etc.; oral infection from feces of animals such as dogs and cats; oral infection from saltwater fish, freshwater fish, crustaceans, shellfish, raw meat of domestic animals, etc.; infection from mosquitoes, gadflies, flies, cockroaches, mites and ticks, fleas, lice, assassin bugs, trombiculid mites, etc.; and the like can be prevented from occurring.

The endoparasite control agent of the present invention can be administered as a pharmaceutical for treatment or prevention of parasitosis in humans and animals of non-human mammalian or avian species. The mode of administration may be oral or parenteral administration. In the case of oral administration, the endoparasite control agent of the present invention can be administered, for example, as a capsule, a tablet, a pill, a powder, a granule, a fine granule, a powder, a syrup, an enteric-coated preparation, a suspension or a paste, or after blended in a liquid drink or feed for animals. In the case of parenteral administration, the endoparasite control agent of the present invention can be administered, for example, as an injection, an infusion, a suppository, an emulsion, a suspension, a drop, an ointment, a cream, a solution, a lotion, a spray, an aerosol, a cataplasm or a tape, or in a dosage form which allows sustained mucosal or percutaneous absorption.

In the case where the endoparasite control agent of the present invention is used as a pharmaceutical for humans and animals of non-human mammalian or avian species, the optimum amount (effective amount) of the active ingredient varies with the purpose (treatment or prevention), the kind of infectious parasite, the type and severity of infection, the dosage form, etc., but in general, the oral daily dose is in the range of about 0.0001 to 10000 mg/kg body weight and the parenteral daily dose is in the range of about 0.0001 to 10000 mg/kg body weight. Such a dose may be given as a single dose or divided into multiple doses.

The concentration of the active ingredient in the endoparasite control agent of the present invention is generally about 0.001 to 100% by mass, preferably about 0.001 to 99% by mass, and more preferably about 0.005 to 20% by mass. The endoparasite control agent of the present invention may be a composition that can be directly administered, or a highly concentrated composition that needs to be diluted to a suitable concentration before use.

The endoparasite control agent of the present invention can be used in combination with any existing endoparasite control agent for the purpose of reinforcing or complementing its effect. In such a combined use, two or more active ingredients may be mixed and formulated into a single preparation before administration, or two or more different preparations may be administered separately.

### EXAMPLES

Next, the present invention will be illustrated in detail by formulation examples and test example of the endoparasite control agent of the present invention, but the scope of the present invention is not limited by the following formulation examples and test example.

In Examples, "part" means a part by mass.

### Formulation Example 1 (emulsion)

Ten parts of a heterocyclic carboxamide derivative represented by the general formula (I) of the present invention, 6 parts of Sorpol 355S (surfactant, manufactured by Toho Chemical Industry), and 84 parts of Solvesso 150 (manufactured by Exxon) are uniformly mixed with stirring to give an emulsion.

### Formulation Example 2 (ointment)

One part of a heterocyclic carboxamide derivative represented by the general formula (I) of the present invention, 50 parts of white beeswax, and 49 parts of white petrolatum are well mixed to give an ointment.

### Formulation Example 3 (tablet)

Two parts of a heterocyclic carboxamide derivative represented by the general formula (I) of the present invention, 10 parts of vegetable oil (olive oil), 3 parts of crystalline cellulose, 20 parts of white carbon, and 65 parts of kaolin are well mixed and compressed into a tablet.

### Formulation Example 4 (injection)

Ten parts of a heterocyclic carboxamide derivative represented by the general formula (I) of the present invention, 10 parts of propylene glycol for use as a food additive, and 80 parts of vegetable oil (corn oil) are mixed to give an injection.

### Formulation Example 5 (solution)

Five parts of a heterocyclic carboxamide derivative represented by the general formula (I) of the present invention, 20 parts of a surfactant for ordinary use as a dissolution or suspension aid, and 75 parts of ion exchanged water are well mixed to give a solution.

### Test Example

### Test for Effect on Motion of Larvae of Haemonchus Nematode (Haemonchus contortus)

The compound of the present invention was prepared as solutions in 100% DMSO at the final concentrations of 50 ppm, 5 ppm, 0.5 ppm, 0.05 ppm and 0.005 ppm. DMSO stands for dimethyl sulfoxide.

A larval suspension containing 1st-stage larvae of *Haemonchus contortus* harvested by the Baermann technique (for example, see K. Nakazono et al., "Inclination of Baermann funnel wall and efficiency of nematode extraction" Proc. Assoc. Pl. Prot. Kyushu 33: 126-130 (1987)) was placed at a density of 20 larvae per well in a test plate, and 0.5 µL/well of the test solution containing the compound of the present invention diluted to a predetermined concentration was added to the test plate. The plate was kept under the conditions of 27°C/95%RH for 4 days. In the test, ivermectin was used for the positive control and DMSO was used for the negative control.

The motor ability of the larvae was examined with an automatic analyzer equipped with an LCD camera. The inhibitory effect on the motion of the larvae in each treatment plot was corrected based on the inhibitory effect in the plot treated with DMSO only for the negative control. The EC₅₀ value was calculated from the data on the corrected inhibitory effect on the motion of the larvae, and graded according to the criteria shown below.

The test was conducted in duplicate per plot.

### Grading criteria

EC₅₀ value:

| | |
|---|---|
| 0.05 ppm or less | A |
| 0.05 to 1 ppm | B |
| 1 to 10 ppm | C |
| 10 ppm or more | D |

As a result, the compounds 1-2, 1-3, 1-4, 1-5, 1-6, 1-7, 1-8, 1-10, 1-11, 1-12, 1-13, 1-14, 1-15, 1-16, 1-17, 1-18, 1-19, 1-21, 1-22, 1-23, 1-24, 1-25, 1-26, 1-27, 1-28, 1-29, 1-30, 1-31, 1-32, 1-33, 1-34, 1-35, 1-36, 1-37, 1-38, 1-39, 1-40, 1-41, 1-42, 1-43, 1-44 and 1-45 of the present invention showed the activity level graded as C or higher. The results show that the compounds of the present invention are effective as an endoparasite control agent.

## Claims

1. An agent for use in the control of endoparasite in animals, said agent comprising, as an active ingredient, a heterocyclic carboxamide derivative represented by the general formula (I): 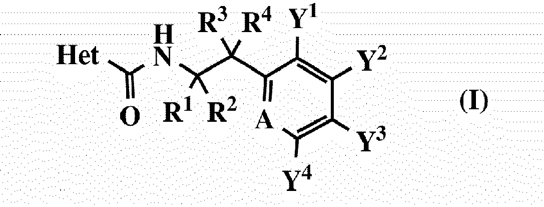 {wherein
R¹ and R² may be the same or different, and are selected from the group consisting of
(a1) a hydrogen atom;
(a2) a (C₁-C₆) alkyl group; and
(a3) a (C₁-C₆) alkoxy group, or optionally
(a4) R¹ and R² together with the carbon atom bound to R¹ and R² form a (C₃-C₆) cycloalkane,
R³ and R⁴ may be the same or different, and are selected from the group consisting of
(b1) a hydrogen atom;
(b2) a (C₁-C₆) alkyl group; and
(b3) a (C₁-C₆) alkoxy group, or optionally
(b4) R³ and R⁴ together with the carbon atom bound to R³ and R⁴ form a (C₃-C₆) cycloalkane,
Y¹ is
(c1) a hydrogen atom;
(c2) a halogen atom;
(c3) a cyano group;
(c4) a nitro group;
(c5) a (C₁-C₆) alkyl group;
(c6) a (C₁-C₆) alkoxy group;
(c7) a halo (C₁-C₆) alkyl group; or
(c8) a halo (C₁-C₆) alkoxy group,
Y² and Y⁴ may be the same or different, and are selected from the group consisting of
(d1) a hydrogen atom;
(d2) a halogen atom;
(d3) a (C₁-C₆) alkyl group;
(d4) a (C₁-C₆) alkoxy group;
(d5) a halo (C₁-C₆) alkyl group; and
(d6) a halo (C₁-C₆) alkoxy group,
Y³ is selected from the group consisting of
(e1) a phenyl group;
(e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
(e3) a phenoxy group;
(e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e5) a pyridyl group;
(e6) a pyridyl group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e7) a pyridyloxy group;
(e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
(e9) a pyrimidyloxy group;
(e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e11) a pyrazyloxy group;
(e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e13) a pyrazolyloxy group;
(e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
(e15) a quinolyloxy group;
(e16) a quinolyloxy group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e17) a quinoxalyloxy group;
(e18) a quinoxalyloxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e19) a benzoxazolyloxy group;
(e20) a benzoxazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e21) a benzothiazolyloxy group;
(e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e23) a furanyl group;
(e24) a furanyl group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e25) a thienyl group;
(e26) a thienyl group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e27) a naphthyl group;
(e28) a naphthyl group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e29) a naphthyloxy group; and
(e30) a naphthyloxy group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group,
A is a nitrogen atom or a CZ group (wherein Z represents a hydrogen atom, (a) a halogen atom, (d) a (C₁-C₆) alkyl group, (e) a halo (C₁-C₆) alkyl group, (f) a (C₁-C₆) alkoxy group or (g) a halo (C₁-C₆) alkoxy group), and
Het represents 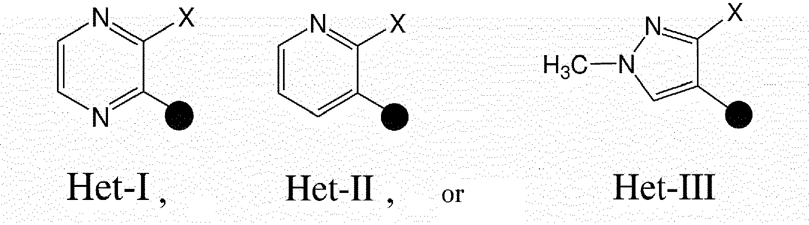 (wherein X represents a halogen atom or a halo (C₁-C₆) alkyl group, and the black circle represents a binding site)}, or a salt thereof.

2. The agent for use according to claim 1, wherein
R¹ and R² are (a1) hydrogen atoms,
R³ and R⁴ may be the same or different, and are selected from the group consisting of
(b1) a hydrogen atom;
(b2) a (C₁-C₆) alkyl group; and
(b3) a (C₁-C₆) alkoxy group, or optionally
(b4) R³ and R⁴ together with the carbon atom bound to R³ and R⁴ form a (C₃-C₆) cycloalkane,
Y¹ is (c1) a halogen atom,
Y² and Y⁴ are (d1) hydrogen atoms, and
Y³ is selected from the group consisting of
(e1) a phenyl group;
(e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
(e3) a phenoxy group;
(e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e5) a pyridyl group;
(e6) a pyridyl group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e7) a pyridyloxy group;
(e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
(e9) a pyrimidyloxy group;
(e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e11) a pyrazyloxy group;
(e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e13) a pyrazolyloxy group;
(e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
(e15) a quinolyloxy group;
(e16) a quinolyloxy group having, on the ring, 1 to 6 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e17) a quinoxalyloxy group;
(e18) a quinoxalyloxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e19) a benzoxazolyloxy group;
(e20) a benzoxazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e21) a benzothiazolyloxy group; and
(e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group.

3. The agent for use according to claim 1, wherein
R¹ and R² are (a1) hydrogen atoms,
R³ and R⁴ may be the same or different, and are selected from the group consisting of
(b1) a hydrogen atom;
(b2) a (C₁-C₆) alkyl group; and
(b3) a (C₁-C₆) alkoxy group, or optionally
(b4) R³ and R⁴ together with the carbon atom bound to R³ and R⁴ form a (C₃-C₆) cycloalkane,
Y¹ is (c1) a halogen atom,
Y² and Y⁴ are (d1) hydrogen atoms,
Y³ is selected from the group consisting of
(e1) a phenyl group;
(e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
(e3) a phenoxy group;
(e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e5) a pyridyl group;
(e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
(e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e11) a pyrazyloxy group;
(e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
(e15) a quinolyloxy group;
(e17) a quinoxalyloxy group;
(e19) a benzoxazolyloxy group;
(e21) a benzothiazolyloxy group; and
(e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group, and
Het is Het-I.

4. A pyrazine carboxamide derivative represented by the general formula (I): 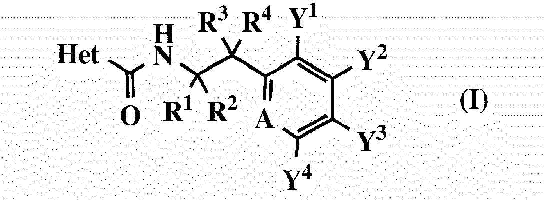 {wherein
R¹ and R² are (a1) hydrogen atoms,
R³ and R⁴ may be the same or different, and are selected from the group consisting of
(b1) a hydrogen atom;
(b2) a (C₁-C₆) alkyl group; and
(b3) a (C₁-C₆) alkoxy group, or optionally
(b4) R³ and R⁴ together with the carbon atom bound to R³ and R⁴ form a (C₃-C₆) cycloalkane,
Y¹ is (c1) a halogen atom,
Y² and Y⁴ are (d1) hydrogen atoms,
Y³ is selected from the group consisting of
(e1) a phenyl group;
(e2) a phenyl group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group and (f) a (C₁-C₆) alkoxy group;
(e3) a phenoxy group;
(e4) a phenoxy group having, on the ring, 1 to 5 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e5) a pyridyl group;
(e8) a pyridyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (f) a (C₁-C₆) alkoxy group and (g) a halo (C₁-C₆) alkoxy group;
(e10) a pyrimidyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e11) a pyrazyloxy group;
(e12) a pyrazyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group;
(e14) a pyrazolyloxy group having, on the ring, 1 to 3 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group, (g) a halo (C₁-C₆) alkoxy group and (h) a formyl group;
(e15) a quinolyloxy group;
(e17) a quinoxalyloxy group;
(e19) a benzoxazolyloxy group;
(e21) a benzothiazolyloxy group; and
(e22) a benzothiazolyloxy group having, on the ring, 1 to 4 substituting groups which may be the same or different and are selected from the group consisting of (a) a halogen atom, (b) a cyano group, (c) a nitro group, (d) a (C₁-C₆) alkyl group, (e) a halo (C₁-C₆) alkyl group, (f) a (C₁-C₆) alkoxy group and (g) a halo (C₁-C₆) alkoxy group, and
Het represents the following formula Het-I: 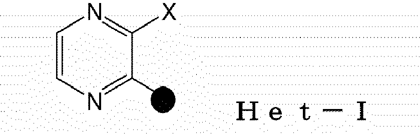 (wherein X represents a halogen atom or a halo (C₁-C₆) alkyl group, and the black circle represents a binding site)}, or a salt thereof.

5. The heterocyclic carboxamide derivative specified in any one of claims 1 to 3 or a salt thereof for use in the control of endoparasites in animals.

## Patentansprüche

1. Agens zur Verwendung bei der Kontrolle von Endoparasiten bei Tieren, wobei das Agens als Wirkstoff ein heterocyclisches Carboxamid-Derivat der allgemeinen Formel (I) umfasst: 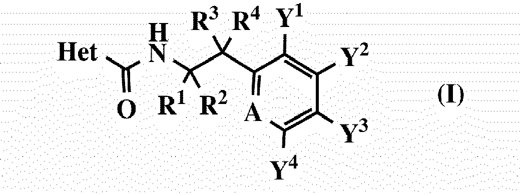 {wobei
R¹ und R² gleich oder verschieden sein können und ausgewählt sind aus der Gruppe bestehend aus
(a1) einem Wasserstoffatom;
(a2) einer (C₁-C₆) Alkylgruppe; und
(a3) einer (C₁-C₆) Alkoxygruppe, oder gegebenenfalls (a4) R¹ und R² zusammen mit dem an R¹ und R² gebundenen Kohlenstoffatom ein (C₃-C₆) Cycloalkan bilden,
R³ und R⁴ können gleich oder verschieden sein und sind ausgewählt aus der Gruppe bestehend aus
(b1) einem Wasserstoffatom;
(b2) einer (C₁-C₆) Alkylgruppe; und
(b3) einer (C₁-C₆) Alkoxygruppe, oder gegebenenfalls
(b4) R³ und R⁴ zusammen mit dem an R³ und R⁴ gebundenen Kohlenstoffatom ein (C₃-C₆) Cycloalkan bilden,
Y¹ ist
(c1) ein Wasserstoffatom;
(c2) ein Halogenatom;
(c3) eine Cyanogruppe;
(c4) eine Nitrogruppe;
(c5) eine (C₁-C₆) Alkylgruppe;
(c6) eine (C₁-C₆) Alkoxygruppe;
(c7) eine Halogen (C₁-C₆) Alkylgruppe; oder
(c8) eine Halogen (C₁-C₆) Alkoxygruppe,
Y² und Y⁴ können gleich oder verschieden sein und sind ausgewählt aus der Gruppe bestehend aus
(d1) einem Wasserstoffatom;
(d2) einem Halogenatom;
(d3) einer (C₁-C₆) Alkylgruppe;
(d4) einer (C₁-C₆) Alkoxygruppe;
(d5) einer Halogen (C₁-C₆) Alkylgruppe; und
(d6) einer Halogen (C₁-C₆) Alkoxygruppe,
Y³ ausgewählt ist aus der Gruppe bestehend aus
(e1) einer Phenylgruppe;
(e2) einer Phenylgruppe mit 1 bis 5 Substitutionsgruppen an dem Ring, die gleich oder verschieden sein können und ausgewählt sind aus der Gruppe, bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe und (f) einer (C₁-C₆) Alkoxygruppe;
(e3) einer Phenoxygruppe;
(e4) einer Phenoxygruppe mit 1 bis 5 Substituentengruppen am Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e5) einer Pyridylgruppe;
(e6) einer Pyridylgruppe mit 1 bis 4 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C1-C6) Alkylgruppe, (e) einer Halogen (C₁-C₆) Alkylgruppe, (f) einer (C₁-C₆)-Alkoxygruppe und (g) einer Halogen (C₁-C₆) Alkoxygruppe;
(e7) einer Pyridyloxygruppe;
(e8) einer Pyridyloxygruppe mit 1 bis 4 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (f) einer (C₁-C₆) Alkoxygruppe und (g) einer Halogen (C₁-C₆) Alkoxygruppe;
(e9) einer Pyrimidyloxygruppe;
(e10) einer Pyrimidyloxygruppe, die an dem Ring 1 bis 3 Substitutionsgruppen aufweist, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e11) einer Pyrazyloxygruppe;
(e12) einer Pyrazyloxygruppe mit 1 bis 3 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆)-Alkylgruppe, (f) einer (C₁-C₆) Alkoxygruppe und (g) einer Halogen (C₁-C₆) Alkoxygruppe;
(e13) einer Pyrazolyloxygruppe;
(e14) einer Pyrazolyloxygruppe mit 1 bis 3 Substitutionsgruppen am Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe, (g) einer Halogen (C₁-C₆)-Alkoxygruppe und (h) einer Formylgruppe;
(e15) einer Chinolyloxygruppe;
(e16) einer Chinolyloxygruppe mit 1 bis 6 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e17) einer Chinoxalyloxygruppe;
(e18) einer Chinoxalyloxygruppe mit 1 bis 5 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e19) einer Benzoxazolyloxygruppe;
(e20) einer Benzoxazolyloxygruppe, die an dem Ring 1 bis 4 Substitutionsgruppen aufweist, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e21) einer Benzothiazolyloxygruppe;
(e22) einer Benzothiazolyloxygruppe mit 1 bis 4 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e23) einer Furanylgruppe;
(e24) einer Furanylgruppe mit 1 bis 3 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e25) einer Thienylgruppe;
(e26) einer Thienylgruppe mit 1 bis 3 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e27) einer Naphthylgruppe;
(e28) einer Naphthylgruppe mit 1 bis 6 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e29) einer Naphthyloxygruppe; und
(e30) einer Naphthyloxygruppe mit 1 bis 6 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe,
A ein Stickstoffatom oder eine CZ-Gruppe ist (worin Z ein Wasserstoffatom, (a) ein Halogenatom, (d) eine (C₁-C₆) Alkylgruppe, (e) eine Halogen (C₁-C₆) Alkylgruppe, (f) eine (C₁-C₆) Alkoxygruppe oder (g) eine Halogen (C₁-C₆) Alkoxygruppe darstellt), und
Het 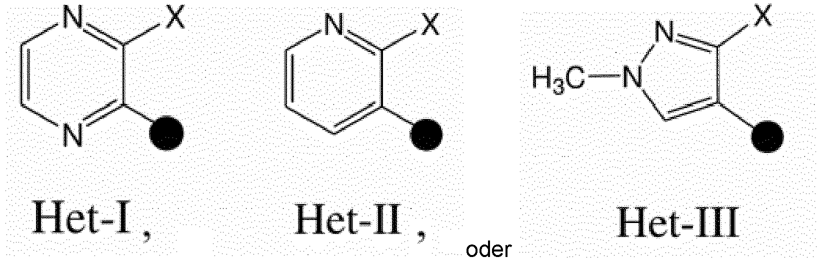 Bedeutet,
(worin X ein Halogenatom oder eine Halogen (C₁-C₆) Alkylgruppe darstellt, und der schwarze Kreis eine Bindungsstelle darstellt)}, oder
ein Salz davon.

2. Angens zur Verwendung nach Anspruch 1, wobei
R¹ und R² (a1) Wasserstoffatome sind,
R³ und R⁴ gleich oder verschieden sein können und ausgewählt sind aus der Gruppe bestehend aus
(b1) einem Wasserstoffatom;
(b2) einer (C₁-C₆) Alkylgruppe; und
(b3) einer (C₁-C₆) Alkoxygruppe, oder gegebenenfalls
(b4) R³ und R⁴ zusammen mit dem an R³ und R⁴ gebundenen Kohlenstoffatom ein (C₃-C₆) Cycloalkan bilden,
Y¹ ist (c1) ein Halogenatom,
Y² und Y⁴ sind (d1) Wasserstoffatome, und
Y³ ausgewählt ist aus der Gruppe bestehend aus
(e1) einer Phenylgruppe;
(e2) einer Phenylgruppe mit 1 bis 5 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe und (f) einer (C₁-C₆) Alkoxygruppe;
(e3) einer Phenoxygruppe;
(e4) einer Phenoxygruppe mit 1 bis 5 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C1-C6) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e5) einer Pyridylgruppe;
(e6) einer Pyridylgruppe mit 1 bis 4 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e7) einer Pyridyloxygruppe;
(e8) einer Pyridyloxygruppe mit 1 bis 4 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (f) einer (C₁-C₆) Alkoxygruppe und (g) einer Halogen (C₁-C₆) Alkoxygruppe;
(e9) einer Pyrimidyloxygruppe;
(e10) einer Pyrimidyloxygruppe mit 1 bis 3 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e11) einer Pyrazyloxygruppe;
(e12) einer Pyrazyloxygruppe mit 1 bis 3 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e13) einer Pyrazolyloxygruppe;
(e14) einer Pyrazolyloxygruppe mit 1 bis 3 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe, (g) einer Halogen (C₁-C₆) Alkoxygruppe und (h) einer Formylgruppe;
(e15) eine Chinolyloxygruppe;
(e16) eine Chinolyloxygruppe mit 1 bis 6 Substitutionsgruppen am Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e17) einer Chinoxalyloxygruppe;
(e18) einer Chinoxalyloxygruppe mit 1 bis 5 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e19) einer Benzoxazolyloxygruppe;
(e20) einer Benzoxazolyloxygruppe mit 1 bis 4 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e21) einer Benzothiazolyloxygruppe; und
(e22) einer Benzothiazolyloxygruppe mit 1 bis 4 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe.

3. Agens zur Verwendung nach Anspruch 1, wobei
R¹ und R² (a1) Wasserstoffatome sind,
R³ und R⁴ gleich oder verschieden sein können und sind ausgewählt aus der Gruppe bestehend aus
(b1) einem Wasserstoffatom;
(b2) einer (C₁-C₆) Alkylgruppe; und
(b3) einer (C₁-C₆) Alkoxygruppe, oder gegebenenfalls
(b4) R³ und R4 bilden zusammen mit dem an R³ und R⁴ gebundenen Kohlenstoffatom ein (C₃-C₆) Cycloalkan,
Y¹ ist (c1) ein Halogenatom,
Y² und Y⁴ sind (d1) Wasserstoffatome,
Y³ ausgewählt ist aus der Gruppe bestehend aus
(e1) einer Phenylgruppe;
(e2) einer Phenylgruppe mit 1 bis 5 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe und (f) einer (C₁-C₆) Alkoxygruppe;
(e3) einer Phenoxygruppe;
(e4) einer Phenoxygruppe mit 1 bis 5 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e5) einer Pyridylgruppe;
(e8) einer Pyridyloxygruppe mit 1 bis 4 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (f) einer (C₁-C₆) Alkoxygruppe und (g) einer Halogen (C₁-C₆) Alkoxygruppe;
(e10) einer Pyrimidyloxygruppe mit 1 bis 3 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e11) einer Pyrazyloxygruppe;
(e12) einer Pyrazyloxygruppe mit 1 bis 3 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e14) einer Pyrazolyloxygruppe mit 1 bis 3 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe, (g) einer Halogen (C₁-C₆) Alkoxygruppe und (h) einer Formylgruppe;
(e15) einer Chinolyloxygruppe;
(e17) einer Chinoxalyloxygruppe;
(e19) einer Benzoxazolyloxygruppe;
(e21) einer Benzothiazolyloxygruppe; und
(e22) einer Benzothiazolyloxygruppe mit 1 bis 4 Substituentengruppen an dem Ring, die gleich oder verschieden sein können und ausgewählt sind aus der Gruppe bestehend aus (a) einem Halogengenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe, und
Het ist Het-I.

4. Pyrazin-Carboxamid-Derivat, dargestellt durch die allgemeine Formel (I): 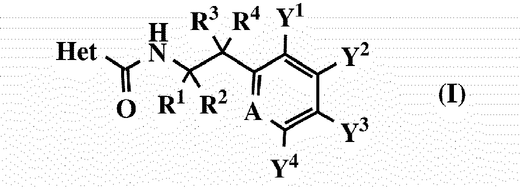 {worin
R¹ und R² (a1) Wasserstoffatome sind,
R³ und R⁴ gleich oder verschieden sein können und sind ausgewählt aus der Gruppe bestehend aus
(b1) einem Wasserstoffatom;
(b2) einer (C₁-C₆) Alkylgruppe; und
(b3) einer (C₁-C₆) Alkoxygruppe, oder gegebenenfalls
(b4) R3 und R4 bilden zusammen mit dem an R³ und R⁴ gebundenen Kohlenstoffatom ein (C₁-C₆) Cycloalkan,
Y¹ ist (c1) ein Halogenatom,
Y² und Y⁴ sind (d1) Wasserstoffatome,
Y³ ausgewählt ist aus der Gruppe bestehend aus
(e1) einer Phenylgruppe;
(e2) einer Phenylgruppe mit 1 bis 5 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe und (f) einer (C₁-C₆) Alkoxygruppe;
(e3) einer Phenoxygruppe;
(e4) einer Phenoxygruppe mit 1 bis 5 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e5) einer Pyridylgruppe;
(e8) einer Pyridyloxygruppe mit 1 bis 4 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (f) einer (C₁-C₆) Alkoxygruppe und (g) einer Halogen (C₁-C₆) Alkoxygruppe; (e10) einer Pyrimidyloxygruppe mit 1 bis 3 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e11) einer Pyrazyloxygruppe;
(e12) einer Pyrazyloxygruppe mit 1 bis 3 Substituentengruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe;
(e14) einer Pyrazolyloxygruppe mit 1 bis 3 Substitutionsgruppen an dem Ring, die gleich oder verschieden sein können und ausgewählt sind aus der Gruppe bestehend aus (a) einem Halogenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe, (g) einer Halogen (C₁-C₆) Alkoxygruppe und (h) einer Formylgruppe;
(e15) einer Chinolyloxygruppe;
(e17) einer Chinoxalyloxygruppe;
(e19) einer Benzoxazolyloxygruppe;
(e21) einer Benzothiazolyloxygruppe; und
(e22) einer Benzothiazolyloxygruppe mit 1 bis 4 Substituentengruppen an der Ring, die gleich oder verschieden sein können und ausgewählt sind aus der Gruppe bestehend aus (a) einem Halogengenatom, (b) einer Cyanogruppe, (c) einer Nitrogruppe, (d) einer (C₁-C₆) Alkylgruppe, (e) einer Halogen (C₁-C₆) Alkylgruppe, (f) einer (C₁-C₆) Alkoxygruppe und (g) einer Halogen (C₁-C₆) Alkoxygruppe, und
Het die folgende Formel Het-I darstellt: 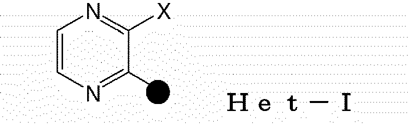 (worin X ein Halogenatom oder eine Halogen (C₁-C₆) Alkylgruppe darstellt, und der schwarze Kreis eine Bindungsstelle darstellt)}, oder
ein Salz davon.

5. Heterocyclisches Carboxamid-Derivat, spezifiziert in einem der Ansprüche 1 bis 3 oder ein Salz davon zur Verwendung bei der Bekämpfung von Endoparasiten bei Tieren.

## Revendications

1. Agent pour une utilisation dans la lutte contre des endoparasites chez les animaux, ledit agent comprenant, en tant qu'ingrédient actif, un dérivé de carboxamide hétérocyclique représenté par la formule générale (I) : 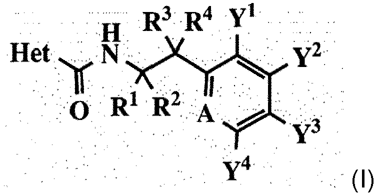 {dans laquelle
R¹ et R² peuvent être identiques ou différents, et sont choisis dans le groupe consistant en
(a1) un atome d'hydrogène ;
(a2) un groupe alkyle en (C₁ à C₆) ; et
(a3) un groupe alcoxy en (C₁ à C₆), ou éventuellement
(a4) R¹ et R² ensemble avec l'atome de carbone lié à R¹ et R² forment un cycloalcane en (C₃ à C₆),
R³ et R⁴ peuvent être identiques ou différents, et sont choisis dans le groupe consistant en
(b1) un atome d'hydrogène ;
(b2) un groupe alkyle en (C₁ à C₆) ; et
(b3) un groupe alcoxy en (C₁ à C₆), ou éventuellement
(b4) R³ et R⁴ ensemble avec l'atome de carbone lié à R³ et R⁴ forment un cycloalcane en (C₃ à C₆),
Y¹ est
(c1) un atome d'hydrogène ;
(c2) un atome d'halogène ;
(c3) un groupe cyano ;
(c4) un groupe nitro ;
(c5) un groupe alkyle en (C₁ à C₆) ;
(c6) un groupe alcoxy en (C₁ à C₆) ;
(c7) un groupe halogénoalkyle en (C₁ à C₆) ; ou
(c8) un groupe halogénoalcoxy en (C₁ à C₆),
Y² et Y⁴ peuvent être identiques ou différents et sont choisis dans le groupe consistant en
(d1) un atome d'hydrogène ;
(d2) un atome d'halogène ;
(d3) un groupe alkyle en (C₁ à C₆) ;
(d4) un groupe alcoxy en (C₁ à C₆) ;
(d5) un groupe halogénoalkyle en (C₁ à C₆) ; et
(d6) un groupe halogénoalcoxy en (C₁ à C₆),
Y³ est choisi dans le groupe consistant en
(e1) un groupe phényle ;
(e2) un groupe phényle ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆) et (f) un groupe alcoxy en (C₁ à C₆) ;
(e3) un groupe phénoxy ;
(e4) un groupe phénoxy ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e5) un groupe pyridyle ;
(e6) un groupe pyridyle ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e7) un groupe pyridyloxy ;
(e8) un groupe pyridyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (f) un groupe alcoxy en (C₁ à C₆) et (g) un groupe halogénoalcoxy en (C₁ à C₆) ;
(e9) un groupe pyrimidyloxy ;
(e10) un groupe pyrimidyloxy ayant, sur le cycle, 1 à 3 groupes substituants qui peuvent être identiques ou différents et qui sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e11) un groupe pyrazyloxy ;
(e12) un groupe pyrazyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e13) un groupe pyrazolyloxy ;
(e14) un groupe pyrazolyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) , (g) un groupe halogénoalcoxy en (C₁ à C₆) et (h) un groupe formyle ;
(e15) un groupe quinolyloxy ;
(e16) un groupe quinolyloxy ayant, sur le cycle, 1 à 6 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e17) un groupe quinoxalyloxy ;
(e18) un groupe quinoxalyloxy ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e19) un groupe benzoxazolyloxy ;
(e20) un groupe benzoxazolyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e21) un groupe benzothiazolyloxy ;
(e22) un groupe benzothiazolyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e23) un groupe furanyle ;
(e24) un groupe furanyle ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e25) un groupe thiényle ;
(e26) un groupe thiényle ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e27) un groupe naphtyle ;
(e28) un groupe naphtyle ayant, sur le cycle, 1 à 6 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e29) un groupe naphtyloxy ; et
(e30) un groupe naphtyloxy ayant, sur le cycle, 1 à 6 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
A est un atome d'azote ou un groupe CZ (où Z représente un atome d'hydrogène, (a) un atome d'halogène, (d) un groupe alkyle en (C₁ à C₆), (e) un groupe halogénoalkyle en (C₁ à C₆), (f) un groupe alcoxy en (C₁ à C₆) ou (g) un groupe halogénoalcoxy en (C₁ à C₆), et
Het représente 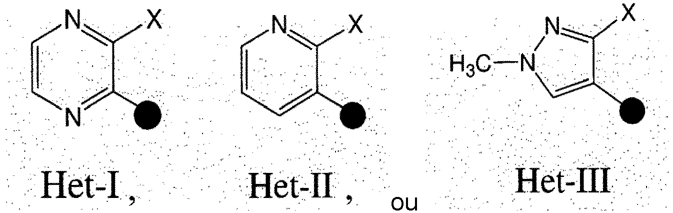 (où X représente un atome d'halogène ou un groupe halogénoalkyle en (C₁ à C₆), et le cercle noir représente un site de liaison)}, ou un sel de celui-ci.

2. Agent pour une utilisation selon la revendication 1, dans lequel
R¹ et R² sont (a1) des atomes d'hydrogène,
R³ et R⁴ peuvent être identiques ou différents et sont choisis dans le groupe consistant en
(b1) un atome d'hydrogène ;
(b2) un groupe alkyle en (C₁ à C₆) ; et
(b3) un groupe alcoxy en (C₁ à C₆), ou éventuellement
(b4) R³ et R⁴ ensemble avec l'atome de carbone lié à R³ et R⁴ forment un cycloalcane en (C₃ à C₆),
Y¹ est (c1) un atome d'halogène ;
Y² et Y⁴ sont (d1) des atomes d'hydrogène, et
Y³ est choisi dans le groupe consistant en
(e1) un groupe phényle ;
(e2) un groupe phényle ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆) et (f) un groupe alcoxy en (C₁ à C₆) ;
(e3) un groupe phénoxy ;
(e4) un groupe phénoxy ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e5) un groupe pyridyle ;
(e6) un groupe pyridyle ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e7) un groupe pyridyloxy ;
(e8) un groupe pyridyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (f) un groupe alcoxy en (C₁ à C₆) et (g) un groupe halogénoalcoxy en (C₁ à C₆) ;
(e9) un groupe pyrimidyloxy ;
(e10) un groupe pyrimidyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e11) un groupe pyrazyloxy ;
(e12) un groupe pyrazyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e13) un groupe pyrazolyloxy ;
(e14) un groupe pyrazolyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) et (h) un groupe formyle ;
(e15) un groupe quinolyloxy ;
(e16) un groupe quinolyloxy ayant, sur le cycle, 1 à 6 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e17) un groupe quinoxalyloxy ;
(e18) un groupe quinoxalyloxy ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e19) un groupe benzoxazolyloxy ;
(e20) un groupe benzoxazolyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e21) un groupe benzothiazolyloxy ; et
(e22) un groupe benzothiazolyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;

3. Agent pour une utilisation selon la revendication 1, dans lequel
R¹ et R² sont des atomes d'hydrogène (a1),
R³ et R⁴ peuvent être identiques ou différents et sont choisis dans le groupe consistant en
(b1) un atome d'hydrogène ;
(b2) un groupe alkyle en (C₁ à C₆) ; et
(b3) un groupe alcoxy en (C₁ à C₆), ou éventuellement
(b4) R³ et R⁴ ensemble avec l'atome de carbone lié à R³ et R⁴ forment un cycloalcane en (C₃ à C₆),
Y¹ est (c1) un atome d'halogène ;
Y² et Y⁴ sont (d1) des atomes d'hydrogène, et
Y³ est choisi dans le groupe consistant en
(e1) un groupe phényle ;
(e2) un groupe phényle ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆) et (f) un groupe alcoxy en (C₁ à C₆) ;
(e3) un groupe phénoxy ;
(e4) un groupe phénoxy ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e5) un groupe pyridyle ;
(e8) un groupe pyridyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (f) un groupe alcoxy en (C₁ à C₆) et (g) un groupe halogénoalcoxy en (C₁ à C₆) ;
(e10) un groupe pyrimidyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e11) un groupe pyrazyloxy ;
(e12) un groupe pyrazyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e14) un groupe pyrazolyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) , (g) un groupe halogénoalcoxy en (C₁ à C₆) et (h) un groupe formyle ;
(e15) un groupe quinolyloxy ;
(e17) un groupe quinoxalyloxy ;
(e19) un groupe benzoxazolyloxy ;
(e21) un groupe benzothiazolyloxy ; et
(e22) un groupe benzothiazolyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
Het est Het-I.

4. Dérivé de pyrazine carboxamide représenté par la formule générale (I) : 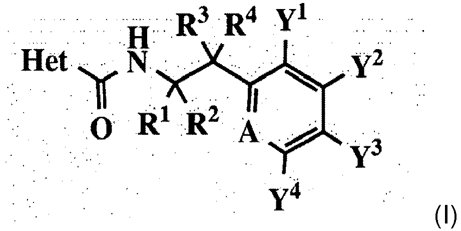 {dans laquelle
R¹ et R² sont (a1) des atomes d'hydrogène,
R³ et R⁴ peuvent être identiques ou différents et sont choisis dans le groupe consistant en
(b1) un atome d'hydrogène ;
(b2) un groupe alkyle en (C₁ à C₆) ; et
(b3) un groupe alcoxy en (C₁ à C₆), ou éventuellement
(b4) R³ et R⁴ ensemble avec l'atome de carbone lié à R³ et R⁴ forment un cycloalcane en (C₃ à C₆),
Y¹ es (c1) un atome d'halogène ;
Y² et Y⁴ sont (d1) des atomes d'hydrogène, et
Y³ est choisi dans le groupe consistant en
(e1) un groupe phényle ;
(e2) un groupe phényle ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆) et (f) un groupe alcoxy en (C₁ à C₆) ;
(e3) un groupe phénoxy ;
(e4) un groupe phénoxy ayant, sur le cycle, 1 à 5 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e5) un groupe pyridyle ;
(e8) un groupe pyridyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (f) un groupe alcoxy en (C₁ à C₆) et (g) un groupe halogénoalcoxy en (C₁ à C₆) ;
(e10) un groupe pyrimidyloxy ayant, sur le cycle, 1 à 3 groupes substituants qui peuvent être identiques ou différents et qui sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e11) un groupe pyrazyloxy ;
(e12) un groupe pyrazyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
(e14) un groupe pyrazolyloxy ayant, sur le cycle, 1 à 3 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) , (g) un groupe halogénoalcoxy en (C₁ à C₆) et (h) un groupe formyle ;
(e15) un groupe quinolyloxy ;
(e17) un groupe quinoxalyloxy ;
(e19) un groupe benzoxazolyloxy ;
(e21) un groupe benzothiazolyloxy ;
(e22) un groupe benzothiazolyloxy ayant, sur le cycle, 1 à 4 groupes substituants qui peuvent être identiques ou différents et sont choisis dans le groupe consistant en (a) un atome d'halogène, (b) un groupe cyano, (c) un groupe nitro, (d) un groupe alkyle en (C₁ à C₆), (e) un groupe halogénoalkyle en (C₁ à C₆), (f) un groupe alcoxy en (C₁ à C₆) et (g) un groupe halogénoalcoxy en (C₁ à C₆) ;
Het représente la formule suivante Het-I : 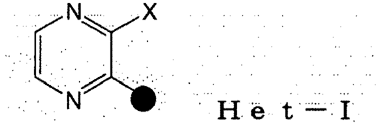 (dans laquelle X représente un atome d'halogène ou un groupe halogénoalkyle en (C₁ à C₆), et le cercle noir représente un site de liaison)}, ou un sel de celui-ci.

5. Dérivé de carboxamide hétérocyclique défini à l'une quelconque des revendications 1 à 3 ou un sel de celui-ci pour une utilisation dans la lutte contre des endoparasites chez les animaux.
